# EUROPEAN PATENT APPLICATION

(11) **EP 4 357 782 A1**
(43) Date of publication of application: **24.04.2024**
(21) Application number: 22202339.2
(22) Date of filing: 18.10.2022
(51) Int. Cl.: G01N 33/574

(54) **PROTEIN BIOMARKER PANEL FOR THE DIAGNOSIS OF COLORECTAL CANCER**

(71) Applicant: MU Bioteknik Aktiebolag, 181 90 Lidingö (SE)
(72) Inventor: UHLÉN, Mathias, 181 90 Lidingö (SE)
(74) Representative: Kransell & Wennborg KB

(57) **Abstract**

There is provided i.a. a method of detecting an increased risk of having or developing colorectal cancer, comprising the steps of:
a) determining the levels of at least proteins PADI4, TFRC, and PRDX5 in a blood sample from a human subject;
b) comparing the determined levels to reference levels; and
c) if the determined levels are higher than the reference levels, concluding that the human subject has the increased risk of having or developing colorectal cancer.

## Description

### TECHNICAL FIELD

The present disclosure relates to the field of cancer diagnostics and in particular to such diagnostics based on detection of proteins in blood-based samples.

### BACKGROUND

A comprehensive characterization of blood proteome profiles in cancer patients can contribute to a better understanding of the disease etiology, resulting in earlier diagnosis, risk stratification and better monitoring of the different cancer subtypes. Cancer Precision Medicine, including the use of molecular tools such as genomics, proteomics and metabolomics, thus aims for a high-resolution individualized diagnosis, with subsequent optimized treatment and monitoring of cancer patients. Of particular importance is the possibility to identify cancers early allowing initiation of treatment and thereby improving patient outcome by avoiding tumor progression, metastasis and emergence of treatment resistant tumors. When cancers are detected at an earlier stage, treatment is more effective and survival is drastically improved (*1*). As an example, according to US-based statistics (*2*), the five-year survival for breast cancer is 99% when detected at an early stage (localized), whereas survival decreases to only 30% when detected at later stages (metastasized). Similarly, the corresponding survival for ovarian cancer is 93% at early stage and 31% when detected at later stage. Based on this, several population screening programs have been initiated to identify cancer before symptoms arise, including screening for prostate cancer using PSA protein level (*3*), colorectal cancer by detecting blood in feces (*4*) and breast cancer using mammography (*5*). However, most population screening tests yield a relatively high number of false positives, causing an unwanted psychological burden for the individual and costly validation before a diagnosis can be confirmed. Moreover, population screening programs are still lacking for the majority of cancers and there is therefore a large need for a single screening test that could detect different forms of cancer at an early stage.

The main focus of Cancer Precision Medicine in the past decade has been to use genomics, involving next generation sequencing to explore the genetic make-up of individual cancers. Huge efforts have been made to gain genetic insight into tumors from patients, including The Cancer Genome Atlas (TCGA) (*6*, *7*); the International Cancer Genome Consortium (ICGC) (8); and the Pan-Cancer Analysis of Whole Genomes (PCAWG) consortium (*9*). Although invaluable insights regarding the biology of individual cancers have been gained by these efforts, the genomics information has not led to any dramatic changes in therapeutic regimes or facilitated screening for cancer in the population. Therefore, a move towards a multi-omics analysis has been suggested (*10*), including functional analysis and alternative assay platforms, such as proteomics using either dissected tumor biopsies or non-invasive body fluids (*11*).

An interesting approach in Cancer Precision Medicine is thus to use protein profiling to allow for liquid biopsy assays from blood. However, the staggering dynamic range in concentrations of blood proteins spanning at least ten orders of magnitude, with concentrations as low as pg/ml for cytokines, makes multiplex analysis involving even a handful of protein targets difficult. This has hampered the development of multiplex blood protein assays during the last few decades. This situation has now changed with the recent development of high-throughput platforms for sensitive proteomics assays in blood, such as Somascan (*12*) and Proximity Extension Assay (PEA) (*13*). These platforms allow thousands of target proteins to be analyzed simultaneously using a few microliters of blood with sensitivity to detect, in a quantitative manner, proteins present in low femtomolar amounts. This means that even proteins well below the detection level for mass spectrometry can now be accurately quantified and used for population screening.

### SUMMARY

The present disclosure based on a novel strategy for pan-cancer analysis in which the plasma profiles of patients with different types of cancer are compared to find cancer-specific signatures that can distinguish each type of cancer from the other cancer types. This is in contrast to the standard procedure for cancer biomarker discovery, in which patients with a specific cancer are compared with healthy controls. Next Generation Blood Profiling (*14*), combining the antibody-based PEA with next generation sequencing, has been used to quantify protein concentrations in multiple cancer types. Samples from more than 1,400 cancer patients from a standardized biobank collection have been analyzed, along with a wealth of clinical meta data (*15*). Altogether, twelve (*12*) cancer types including the most prevalent types such as colorectal-, breast-, lung- and prostate cancer, have been studied. AI-based disease prediction models were used to identify a panel of proteins associated with each of the analyzed cancers. The primary aim of the protein panel is to distinguish one cancer from another. This panel has also subsequently been used to explore the differences in plasma signatures *vis-a-vis* healthy individuals.

Accordingly, the present disclosure provides the following itemized embodiments.
1. A method of detecting an increased risk of having or developing acute myeloid leukemia (AML), comprising the steps of:
   a) determining the levels of at least proteins CD244, TNFSF13B , and FLT3 in a blood sample from a human subject;
   b) comparing the determined levels to reference levels; and
   c) if the determined levels are higher than the reference levels, concluding that the human subject has the increased risk of having or developing AML.
2. The method of item 1, wherein step c) further comprises a further examination of the human subject to establish an AML diagnosis.
3. The method of item 2, wherein the further examination comprises examination of a bone marrow sample from the human subject, typically using a microscope.
4. The method of item 2 or 3, wherein step c) further comprises treating the human subject for AML if an AML diagnosis is established.
5. The method of any one of the preceding items, wherein step a) comprises contacting the blood sample with at least:
   an antibody, antibody fragment or aptamer selective for CD244;
   an antibody, antibody fragment or aptamer selective for TNFSF13B; and
   an antibody, antibody fragment or aptamer selective for FLT3.
6. A method of detecting an increased risk of having or developing chronic lymphocytic leukemia (CLL), comprising the steps of:
   a) determining the levels of at least proteins TCLiA, STC1 and CD22 in a blood sample from a human subject;
   b) comparing the determined levels to reference levels; and
   c) if the determined levels are higher than the reference levels, concluding that the human subject has the increased risk of having or developing CLL.
7. The method of item 6, wherein step c) further comprises a further examination of the human subject to establish an CLL diagnosis.
8. The method of item 7, wherein the further examination comprises an examination of at least one of a bone marrow sample from the human subject, a tissue sample from a lymph gland of the human subject, or a second blood sample from the human subject.
9. The method of item 7 or 8, wherein step c) further comprising treating the human subject for CLL if a CLL diagnosis is established.
10. The method of any one of items 6-9, wherein step a) comprises contacting the blood sample with at least:
   an antibody, antibody fragment or aptamer selective for TCLiA;
   an antibody, antibody fragment or aptamer selective for STCi; and
   an antibody, antibody fragment or aptamer selective for CD22.
11. A method of detecting an increased risk of having or developing diffuse large B-cell lymphoma (DLBCL), comprising the steps of:
   a) determining the levels of at least proteins DCXR, CXCL9, and CXCL13 in a blood sample from a human subject;
   b) comparing the determined levels to reference levels; and
   c) if the determined levels are higher than the reference levels, concluding that the human subject has the increased risk of having or developing DLBCL.
12. The method of item 11, wherein step c) further comprises a further examination of the human subject to establish an DLBCL diagnosis.
13. The method of item 12, wherein the further examination comprises an examination of at least one of a bone marrow sample from the human subject, a tissue sample from a tumour-biopsy on the human subject and at least one image obtained by a CT scan of the human subject.
14. The method of item 12 or 13, wherein step c) further comprising treating the human subject for DLBCL if a DLBCL diagnosis is established.
15. The method of any one of items 11-14, wherein step a) comprises contacting the blood sample with at least:
   an antibody, antibody fragment or aptamer selective for DCXR;
   an antibody, antibody fragment or aptamer selective for CXCL9; and
   an antibody, antibody fragment or aptamer selective for CXCL13.
16. A method of detecting an increased risk of having or developing myeloma, comprising the steps of:
   a) determining the levels of at least proteins CNTN5, SLAMF7, and MZB1 in a blood sample from a human subject;
   b) comparing the determined levels to reference levels; and
   c) if the determined levels are higher than the reference levels, concluding that the human subject has the increased risk of having or developing myeloma.
17. The method of item 16, wherein step c) further comprises a further examination of the human subject to establish a myeloma diagnosis.
18. The method of item 17, wherein the further examination comprises an examination of at least one of a bone marrow sample from the human subject, at least one image obtained by an X-ray of the human subject, a protein electrophoresis of a blood or urine sample from the human subject, or a free light chain quantitation on serum from a human subject.
19. The method of item 17 or 18, wherein step c) further comprising treating the human subject for myeloma.
20. The method of any one of items 16-19, wherein step a) comprises contacting the blood sample with at least:
   an antibody, antibody fragment or aptamer selective for CNTN5;
   an antibody, antibody fragment or aptamer selective for SLAMF7; and
   an antibody, antibody fragment or aptamer selective for MZB1.
21. A method of detecting an increased risk of having or developing colorectal cancer, comprising the steps of:
   a) determining the levels of at least proteins PADI4, TFRC, and PRDX5 in a blood sample from a human subject;
   b) comparing the determined levels to reference levels; and
   c) if the determined levels are higher than the reference levels, concluding that the human subject has the increased risk of having or developing colorectal cancer.
22. The method of item 21, wherein step c) further comprises a further examination of the human subject to establish a colorectal cancer diagnosis.
23. The method of item 22, wherein the further examination comprises at least one of a proctoscopy on the human subject, a rectoscopy on the human subject, or an examination of a tissue sample from a tumour-biopsy on the human subject.
24. The method of item 22 or 23, wherein step c) further comprising treating the human subject for colorectal cancer if a colorectal cancer diagnosis is established.
25. The method of any one of items 21-24, wherein step a) comprises contacting the blood sample with at least:
   an antibody, antibody fragment or aptamer selective for PADI4;
   an antibody, antibody fragment or aptamer selective for TFRC; and
   an antibody, antibody fragment or aptamer selective for PRDX5.
26. A method of detecting an increased risk of having or developing lung cancer, comprising the steps of:
   a) determining the levels of at least proteins BCL2L11, MMP12, and CEACAM5 in a blood sample from a human subject;
   b) comparing the determined levels to reference levels; and
   c) if the determined levels are higher than the reference levels, concluding that the human subject has the increased risk of having or developing lung cancer.
27. The method of item 26, wherein step c) further comprises a further examination of the human subject to establish a lung cancer diagnosis.
28. The method of item 27, wherein the further examination comprises at least one of a fiberoptic bronchoscopy on the human subject, an examination of a tissue sample obtained by a tumour-biopsy on the human subject, an examination of at least one image obtained by a CT or PET scan of the lung of the human subject, or an examination of at least one image obtained by an endoscopic ultrasound on the human subject.
29. The method of item 27 or 28, wherein step c) further comprising treating the human subject for lung cancer if a lung cancer diagnosis is established.
30. The method of any one of items 26-29, wherein step a) comprises contacting the blood sample with at least:
   an antibody, antibody fragment or aptamer selective for BCL2L11;
   an antibody, antibody fragment or aptamer selective for MMP12; and
   an antibody, antibody fragment or aptamer selective for CEACAM5.
31. A method of detecting an increased risk of having or developing glioma, comprising the steps of:
   a) determining the levels of at least proteins GFAP, BCAN, and ADAMTS13 in a blood sample from a human subject;
   b) comparing the determined levels to reference levels; and
   c) if the determined levels are higher than the reference levels, concluding that the human subject has the increased risk of having or developing glioma.
32. The method of item 31, wherein step c) further comprises a further examination of the human subject to establish a glioma diagnosis.
33. The method of item 32, wherein the further examination comprises an examination of at least one image obtained by a CT scan of the brain of the human subject.
34. The method of item 32 or 33, wherein step c) further comprising treating the human subject for glioma if a glioma diagnosis is established.
35. The method of any one of items 31-34, wherein step a) comprises contacting the blood sample with at least:
   an antibody, antibody fragment or aptamer selective for GFAP;
   an antibody, antibody fragment or aptamer selective for BCAN; and
   an antibody, antibody fragment or aptamer selective for ADAMTS13.
36. A method of detecting an increased risk of having or developing breast cancer, comprising the steps of:
   a) determining the levels of at least proteins PRTG, LAMP3, and OXT in a blood sample from a human subject;
   b) comparing the determined levels to reference levels; and
   c) if the determined levels are higher than the reference levels, concluding that the human subject has the increased risk of having or developing breast cancer.
37. The method of item 36, wherein step c) further comprises a further examination of the human subject to establish a breast cancer diagnosis.
38. The method of item 37, wherein the further examination comprises an examination of at least one of a tissue sample from a tumour-biopsy on the human subject, or at least one image obtained by a mammography on the human subject.
39. The method of item 37 or 38, wherein step c) further comprising treating the human subject for breast cancer if a breast cancer diagnosis is established.
40. The method of any one of items 37-39, wherein step a) comprises contacting the blood sample with at least:
   an antibody, antibody fragment or aptamer selective for PRTG;
   an antibody, antibody fragment or aptamer selective for LAMP3; and
   an antibody, antibody fragment or aptamer selective for OXT.
41. A method of detecting an increased risk of having or developing cervical cancer, comprising the steps of:
   a) determining the levels of at least proteins GLO1, CHRDL2, and FCGR3B in a blood sample from a human subject;
   b) comparing the determined levels to reference levels; and
   c) if the determined levels are higher than the reference levels, concluding that the human subject has the increased risk of having or developing cervical cancer.
42. The method of item 41, wherein step c) further comprises a further examination of the human subject to establish a cervical cancer diagnosis.
43. The method of item 42, wherein the further examination comprises an examination of at least one of a tissue sample from the cervix of the human subject or a pap test from the human subject.
44. The method of item 42 or 43, wherein step c) further comprising treating the human subject for cervical cancer if a cervical cancer diagnosis is established.
45. The method of any one of items 41-44, wherein step a) comprises contacting the blood sample with at least:
   an antibody, antibody fragment or aptamer selective for GLO1;
   an antibody, antibody fragment or aptamer selective for CHRDL2; and
   an antibody, antibody fragment or aptamer selective for FCGR3B.
46. A method of detecting an increased risk of having or developing endometrial cancer, comprising the steps of:
   a) determining the levels of at least proteins TNFSF10, PLAT, and DPT in a blood sample from a human subject;
   b) comparing the determined levels to reference levels; and
   c) if the determined levels are higher than the reference levels, concluding that the human subject has the increased risk of having or developing endometrial cancer.
47. The method of item 46, wherein step c) further comprises a further examination of the human subject to establish an endometrial cancer diagnosis.
48. The method of item 47, wherein the further examination comprises an examination of at least one of a tissue sample from the endometrium of the human subject, or a tissue sample obtained by scraping the uterus and/or cervix of the human subject.
49. The method of item 47 or 48, wherein step c) further comprising treating the human subject for endometrial cancer if an endometrial cancer diagnosis is established.
50. The method of any one of items 46-49, wherein step a) comprises contacting the blood sample with at least:
   an antibody, antibody fragment or aptamer selective for TNFSF10;
   an antibody, antibody fragment or aptamer selective for PLAT; and
   an antibody, antibody fragment or aptamer selective for DPT.
51. A method of detecting an increased risk of having or developing ovarian cancer, comprising the steps of:
   a) determining the levels of at least proteins PAEP, CDH3, and SSC5D in a blood sample from a human subject;
   b) comparing the determined levels to reference levels; and
   c) if the determined levels are higher than the reference levels, concluding that the human subject has the increased risk of having or developing ovarian cancer.
52. The method of item 51, wherein step c) further comprises a further examination of the human subject to establish an ovarian cancer diagnosis.
53. The method of item 52, wherein the further examination comprises an examination of at least one image obtained from a pelvic ultrasound of the human subject, a second blood sample from the human subject, or at least one image obtained by a CT scan of the human subject.
54. The method of item 52 or 53, wherein step c) further comprising treating the human subject for ovarian cancer if an ovarian cancer diagnosis is established.
55. The method of any one of items 51-54, wherein step a) comprises contacting the blood sample with at least:
   an antibody, antibody fragment or aptamer selective for PAEP;
   an antibody, antibody fragment or aptamer selective for CDH3; and
   an antibody, antibody fragment or aptamer selective for SSC5D.
56. A method of detecting an increased risk of having or developing prostate cancer, comprising the steps of:
   a) determining the levels of at least proteins FAP, DNER, and IL20 in a blood sample from a human subject;
   b) comparing the determined levels to reference levels; and
   c) if the determined levels are higher than the reference levels, concluding that the human subject has the increased risk of having or developing prostate cancer.
57. The method of item 56, wherein step c) further comprises a further examination of the human subject to establish a prostate cancer diagnosis.
58. The method of item 57, wherein the further examination comprises an examination of at least one of a prostate-specific antigen (PSA) test from the human subject, at least one image obtained by a magnetic resonance tomography on the prostate of the human subject, a tissue sample from a tumour-biopsy on the human subject.
59. The method of item 57 or 58, wherein step c) further comprising treating the human subject for prostate cancer if a prostate cancer diagnosis is established.
60. The method of any one of items 56-59, wherein step a) comprises contacting the blood sample with at least:
   an antibody, antibody fragment or aptamer selective for FAP;
   an antibody, antibody fragment or aptamer selective for DNER; and
   an antibody, antibody fragment or aptamer selective for IL20.

### BRIEF DESCRIPTION OF THE DRAWINGS

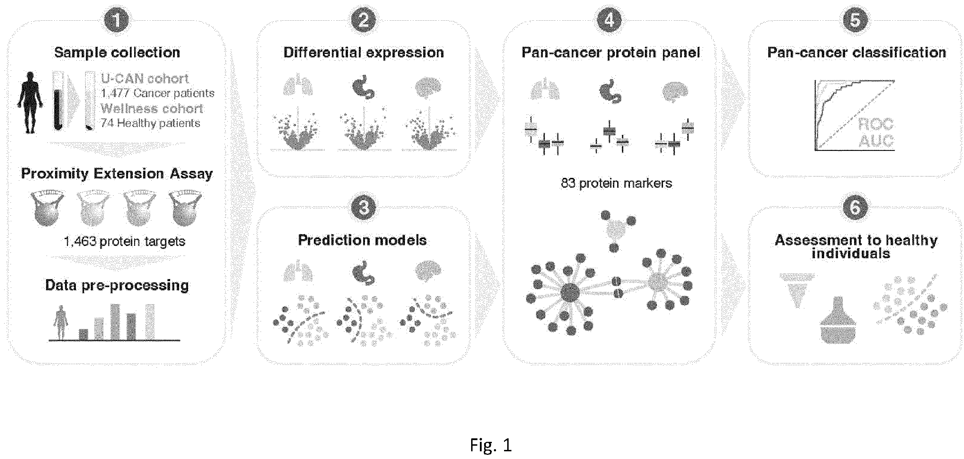

*Fig 1* | *Overview of the pan-cancer study.* Schematic representation of the workflow used to identify a pan-cancer biomarker panel for cancer classification. Blood plasma from 1,477 cancer patients and 74 healthy individuals was analyzed using Proximity Extension Assay. Differential expression analysis and prediction models were performed to identify a pan-cancer protein panel. The model for cancer classification was generated using machine learning techniques (70% of the data). The performance of the resulting pan-cancer protein panel was tested against a model test set (30% of the data) and ultimately compared against healthy individuals.

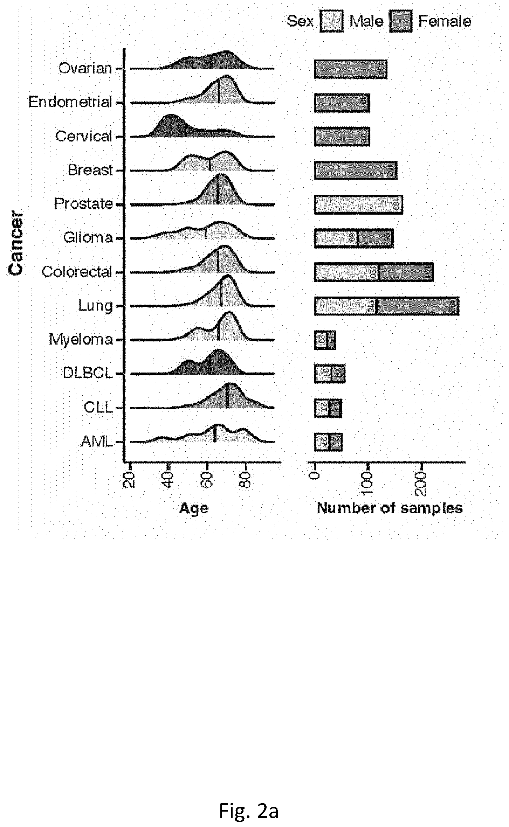

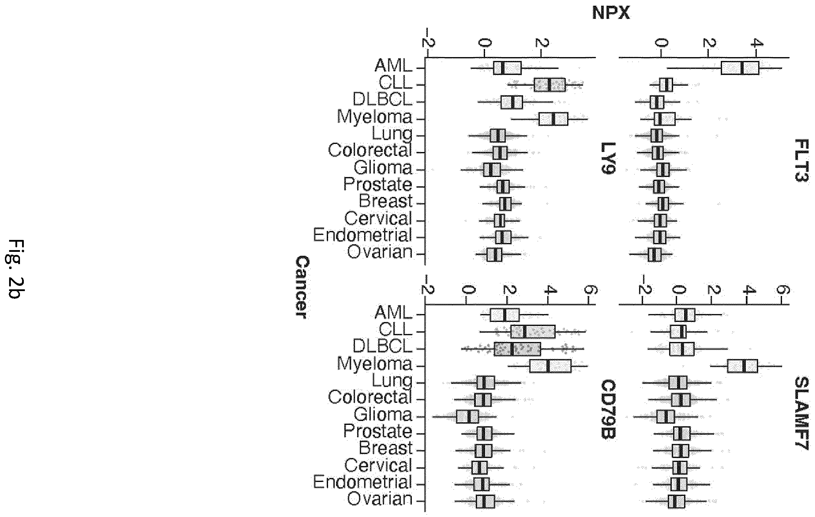

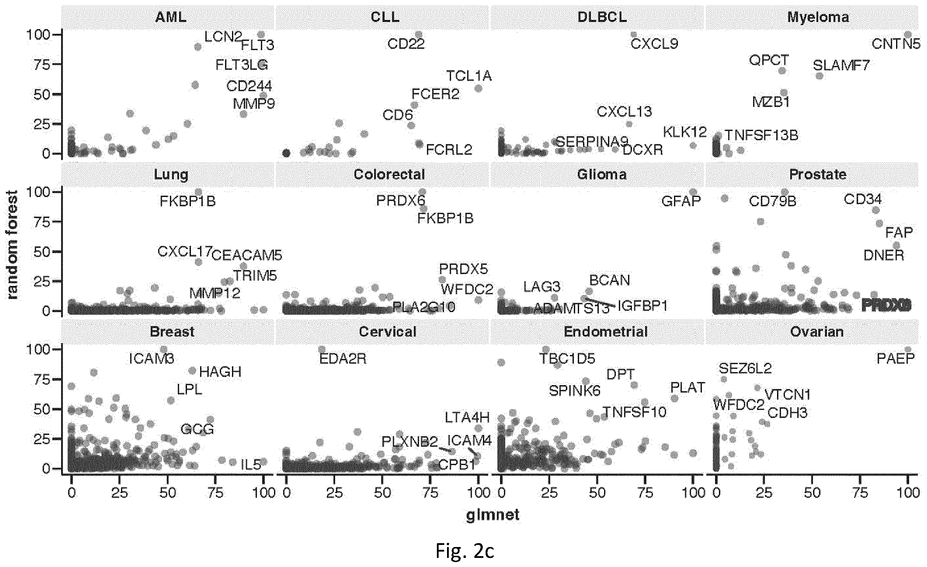

*Fig. 2* | *The study cohort and the prediction models to classify each cancer type.* a, Age distribution and number of patients included for each cancer in the study. b, Examples of protein levels (NPX, y-axis) for four example proteins across the 12 cancer types (x-axis). c, Scatter plot between the importance score for two prediction models (random forest (rf) and a regularized generalized linear model (glmnet)).

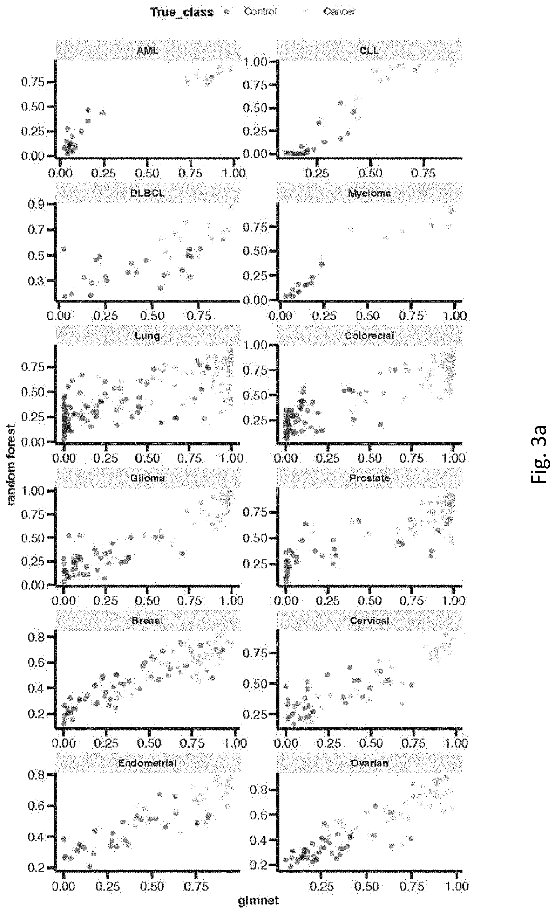

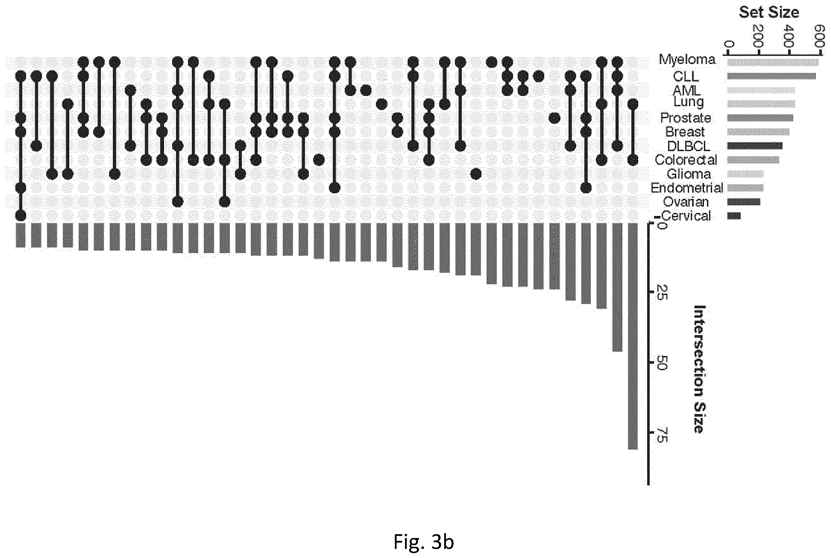

*Fig. 3* | *Performance of prediction models and differential expression analysis.* a, Comparison of cancer probabilities for samples in the test set for glmnet (n=12) and rf (n=12) cancer models. b, Upset plot showing the number of upregulated proteins shared by the different cancer types.

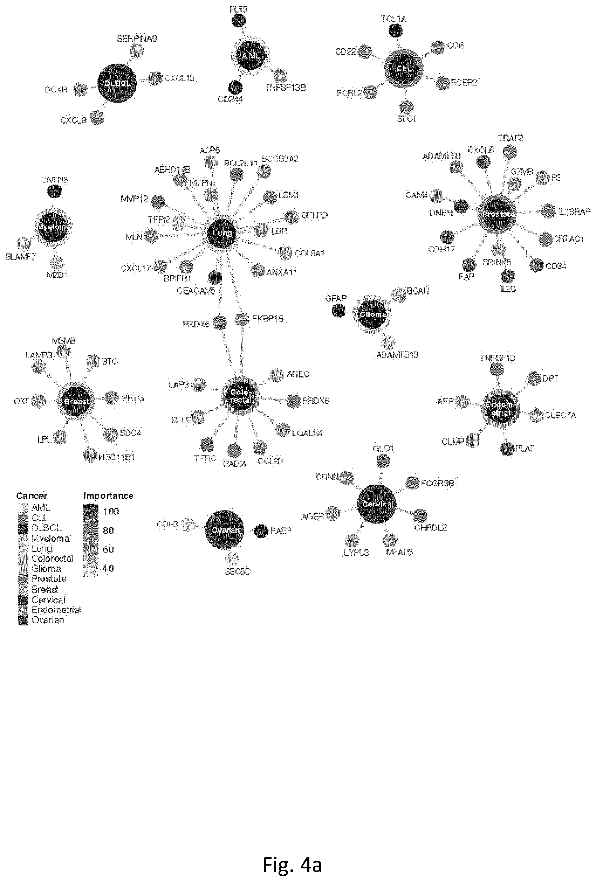

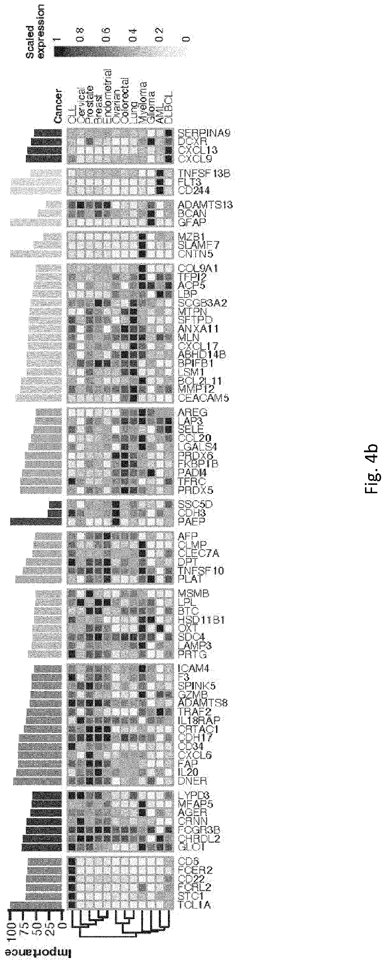

*Fig. 4* | *Pan-cancer protein panel.* a, Network visualization of proteins included in the panel. Protein nodes are colored according to the importance score in the specific cancer. b, Summarized expression profiles of panel proteins across the cancer types.

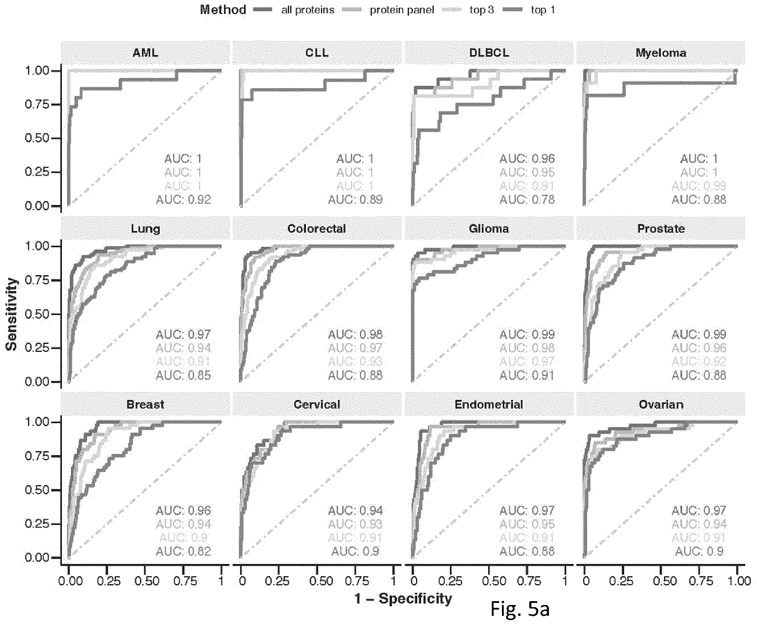

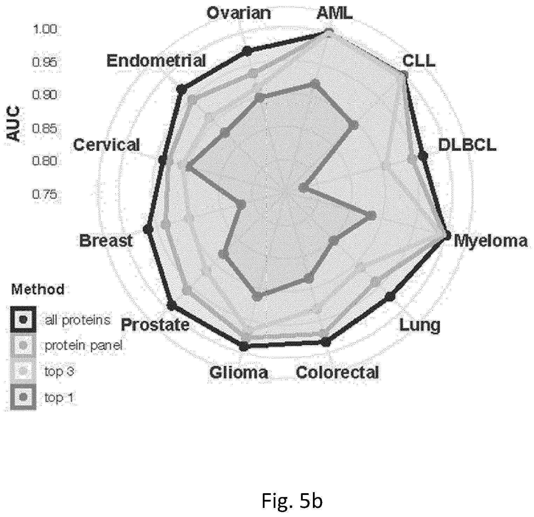

*Fig. 5* | *Multiclassification of the pan-cancer test cohort.* a, ROC curves for the model for the different cancers based on all proteins (n=1,463), the selected protein panel (n=83) and the top 3 (n=3) and the most important protein (n=1) for each of the 12 cancers. b, Summary of the AUC for the different cancers based on different numbers of proteins.

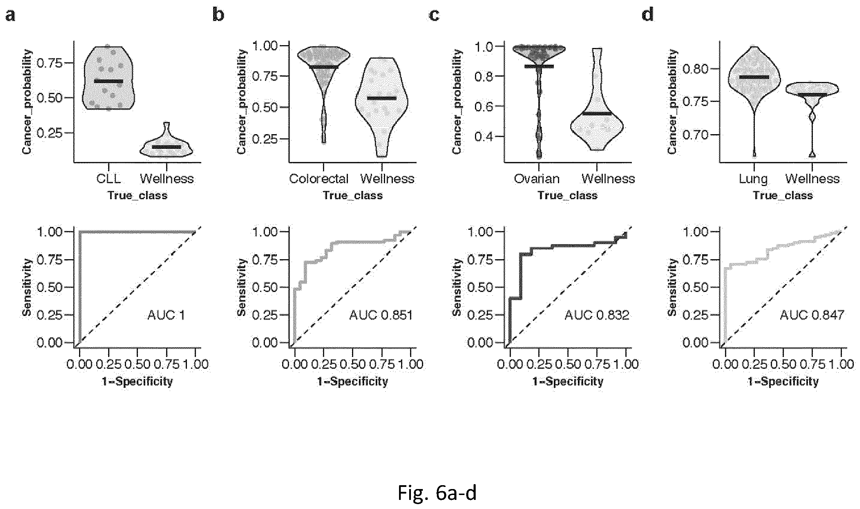

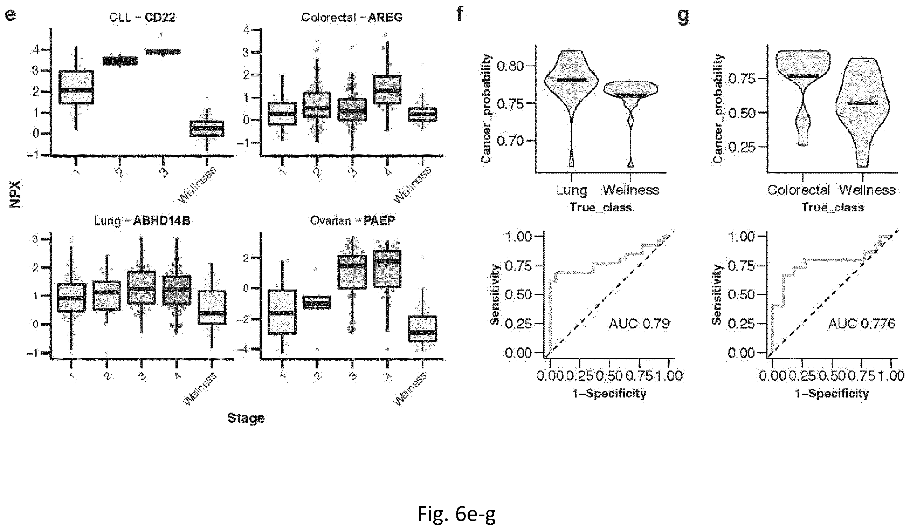

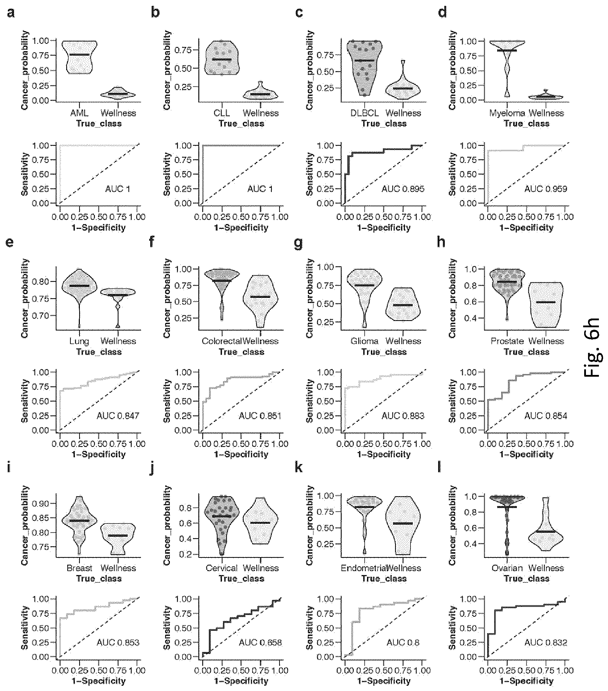

*Fig. 6* | *Classification of cancer samples from a healthy cohort based on the selected protein panel.* Model results showing the cancer probability for cancer and healthy individuals (top) and the ROC curve with AUC score (bottom) for a, CLL, b, colorectal cancer, c, ovarian cancer, d, lung cancer, h, all twelve (12) cancers. e, Protein levels of four different biomarkers across cancer stages. Model results showing the cancer probability for cancer and healthy individuals (top) and the ROC curve with AUC score (bottom) for f, early-stage lung cancer and g, early-stage colorectal cancer.

### DETAILED DESCRIPTION

The present disclosure describes a novel strategy based on next generation plasma profiling to explore the proteome signature in cancer patients by comprehensively exploring the protein levels in a pan-cancer cohort, including patients representing most major cancer types. The assay platform allows thousands of proteins to be quantitatively analyzed using only a few microliters of blood opening up new opportunities for Precision Cancer Medicine. The plasma levels of each individual protein have been determined for more than 1,400 cancer patients representing 12 different cancer types.

Applying AI-based disease prediction models based on all measured proteins allowed us to identify a set of proteins associated with each of the cancers studied. A prediction model based on a restricted set of 83 upregulated proteins was built to evaluate the accuracy of the classification of pan-cancer samples and the analysis showed that each cancer has a distinct plasma proteome profile. The panel allowed the stratification of plasma samples from most cancer types with high sensitivity and specificity in a test cohort based on individual protein levels. The panel was also able to detect, with high accuracy, patients with early disease, exemplified by stage patients in lung and colorectal cancers.

In summary, a novel strategy for exploration of protein profiles in blood to allow simultaneous identification of each of 12 common cancers using only a minute amount (few microliters) of blood is described. Since the assay can be combined with simple sample collection formats, such as dried blood spots, the results open up the possibility for a cost-effective pan-cancer population diagnosis using a panel of proteins to identify most of the common cancers in a single assay. A population screening could be organized to allow the discovery of cancers much earlier and thus help clinicians to start treatment of cancer patients at earlier stages, as compared to today.

As a first aspect of the present disclosure, there is provided a method of detecting an increased risk of having or developing acute myeloid leukemia (AML), comprising the steps of:
a) determining the levels of at least proteins CD244, TNFSF13B, and FLT3 in a blood sample from a human subject;
b) comparing the determined levels to reference levels; and
c) if the determined levels are higher than the reference levels, concluding that the human subject has the increased risk of having or developing AML.

The first aspect can be carried out *ex vivo.*

The reference levels are preferably predetermined in human subjects not suffering from AML, such as healthy human subjects and/or human subjects suffering from another cancer than AML.

The blood sample may for example be a blood plasma sample or a sample derived from dried blood spots.

In one embodiment, step a) of the method of the first aspect comprises contacting the blood sample with at least:
an antibody, antibody fragment or aptamer selective for CD244;
an antibody, antibody fragment or aptamer selective for TNFSF13B ; and
an antibody, antibody fragment or aptamer selective for FLT3.

In one embodiment of the first aspect, step c) further comprises a further examination of the human subject to establish an AML diagnosis. The further examination may for example comprise examination of a bone marrow sample from the human subject, typically using a microscope.

Establishing an AML diagnosis may further include a clinical examination of the human subject including assessment of the general condition of the human subject, wherein symptoms may include anaemia, enlarged lymph glands, fatigue, fever, and susceptibility for infections. The bone marrow sample may be taken from the iliac crest of the human subject. If Auer rods are found in the bone marrow sample an AML diagnosis may be established. The leukemic cells may be further examined to establish a phenotype. Establishing the diagnosis may further comprise at least one of a chromosomal analysis or molecular genetic analysis. Establishing the AML diagnosis may include further steps deemed necessary by a clinician or organisation.

If an AML diagnosis is established, step c) of the method of the first aspect may further comprise treating the human subject for AML.

Treating the human subject for AML may include steps such as administration of cytostatic drugs, a stem cell transplant, a bone marrow transplant, and/or further steps deemed necessary by a clinician.

As a second aspect of the present disclosure, there is provided a method of detecting an increased risk of having or developing chronic lymphocytic leukemia (CLL), comprising the steps of:
a) determining the levels of at least proteins TCLiA, STC1 and CD22 in a blood sample from a human subject;
b) comparing the determined levels to reference levels; and
c) if the determined levels are higher than the reference levels, concluding that the human subject has the increased risk of having or developing CLL.

In one embodiment, step a) of the second aspect further comprises determining the level of at least one (such as at least two) of the proteins FCRL2, FCEL2 and CD6. Accordingly, the determined level of FCRL2, FCEL2, and/or CD6 is compared to a reference level in step b) of this embodiment.

The second aspect can be carried out *ex vivo.*

The reference levels are preferably predetermined in human subjects not suffering from CLL, such as healthy human subjects and/or human subjects suffering from another cancer than CLL.

The blood sample may for example be a blood plasma sample or a sample derived from dried blood spots.

In one embodiment, step a) of the method of the second aspect comprises contacting the blood sample with:
an antibody, antibody fragment or aptamer selective for TCLiA;
an antibody, antibody fragment or aptamer selective for STCi; and
an antibody, antibody fragment or aptamer selective for CD22.

In one embodiment, step a) of the method of the second aspect may further comprise contacting the blood sample with:
an antibody, antibody fragment or aptamer selective for FCRL2;
an antibody, antibody fragment or aptamer selective for FCEL2; and/or
an antibody, antibody fragment or aptamer selective for CD6.

In one embodiment of the second aspect, step c) further comprises a further examination of the human subject to establish an CLL diagnosis. The further examination comprises an examination of at least one of a bone marrow sample from the human subject, a tissue sample from a lymph gland of the human subject, a second blood sample from the human subject, and at least one image obtained by a CT scan of the human subject.

Establishing an CLL diagnosis may further include a clinical examination of the human subject including assessment of the general condition of the human subject, wherein symptoms may include anaemia, enlarged lymph glands, fatigue, fever, susceptibility for infections. A CLL diagnosis may be established if the examination of the second blood sample shows an elevated level of morphologically mature lymphocytes. Flow cytometry may be performed on the leukemic cells to establish a phenotype. Establishing the CLL diagnosis may include further steps deemed necessary by a clinician or organisation.

If an CLL diagnosis is established, step c) of the method of the second aspect may further comprise treating the human subject for CLL.

Treating the human subject for CLL may include steps such as administration of cytostatic drugs, administration of B-cell receptor inhibitor or BCL2-inhibitor, immunotherapy treatment, a stem cell transplant, a bone marrow transplant, and/or further steps deemed necessary by a clinician.

As a third aspect of the present disclosure, there is provided a method of detecting an increased risk of having or developing diffuse large B-cell lymphoma (DLBCL), comprising the steps of:
a) determining the levels of at least proteins DCXR, CXCL9, and CXCL13 in a blood sample from a human subject;
b) comparing the determined levels to reference levels; and
c) if the determined levels are higher than the reference levels, concluding that the human subject has the increased risk of having or developing DLBCL.

In one embodiment, step a) of the third aspect may further comprise determining the level of the protein SERPINA9. Accordingly, the determined level of SERPINA9 is compared to a reference level in step b) of this embodiment. This step may further comprise contacting the blood sample with an antibody, antibody fragment or aptamer selective for SERPINA9.

The third aspect can be carried out *ex vivo.*

The reference levels are preferably predetermined in human subjects not suffering from DLBCL, such as healthy human subjects and/or human subjects suffering from another cancer than DLBCL.

The blood sample may for example be a blood plasma sample or a sample derived from dried blood spots.

In one embodiment, step a) of the method of the third aspect comprises contacting the blood sample with:
an antibody, antibody fragment or aptamer selective for DCXR;
an antibody, antibody fragment or aptamer selective for CXCL9; and
an antibody, antibody fragment or aptamer selective for CXCL13

In one embodiment of the third aspect, step c) further comprises a further examination of the human subject to establish a DLBCL diagnosis. The further examination comprises an examination of at least one of a bone marrow sample from the human subject, a tissue sample from a tumour-biopsy on the human subject and at least one image obtained by a CT scan of the human subject.

Establishing an DLBCL diagnosis may further include a clinical examination of the human subject including assessment of the general condition of the human subject, wherein symptoms may include back pain, enlarged lymph glands, fever and fatigue. An important step in establishing the DLBCL diagnosis is the examination of the tissue sample from the tumour-biopsy on the human subject. The biopsy may be a needle aspiration biopsy or an excisional biopsy. The DLBCL diagnosis may include further steps deemed necessary by a clinician or organisation.

If an DLBCL diagnosis is established, step c) of the method of the third aspect may further comprise treating the human subject for DLBCL.

Treating the human subject for DLBCL may include steps such as administration of cytostatic drugs, a radiation therapy treatment, immunotherapy treatment, a stem cell transplant, CAR-T 19 treatment, and/or further steps deemed necessary by a clinician.

As a fourth aspect of the present disclosure, there is provided a method of detecting an increased risk of having or developing myeloma, comprising the steps of:
a) determining the levels of at least proteins CNTN5, SLAMF7, and MZB1 in a blood sample from a human subject;
b) comparing the determined levels to reference levels; and
c) if the determined levels are higher than the reference levels, concluding that the human subject has the increased risk of having or developing myeloma.

The fourth aspect can be carried out *ex vivo.*

The reference levels are preferably predetermined in human subjects not suffering from myeloma, such as healthy human subjects and/or human subjects suffering from another cancer than myeloma.

The blood sample may for example be a blood plasma sample or a sample derived from dried blood spots.

In one embodiment, step a) of the method of the fourth aspect comprises contacting the blood sample with:
an antibody, antibody fragment or aptamer selective for CNTN5;
an antibody, antibody fragment or aptamer selective for SLAMF7; and
an antibody, antibody fragment or aptamer selective for MZB1.

In one embodiment of the fourth aspect, step c) further comprises a further examination of the human subject to establish a myeloma diagnosis. The further examination comprises a n examination of at least one of a bone marrow sample from the human subject, at least one image obtained by an X-ray of the human subject, a protein electrophoresis of a blood or urine sample from the human subject, or a free light chain quantitation on serum from a human subject.

Establishing a myeloma diagnosis may further include a clinical examination of the human subject including assessment of the general condition of the human subject, wherein the symptoms may include skeletal pain, fatigue, anaemia, fever, susceptibility for infections, hypercalcemia, and neurological symptoms. The X-ray images may be of the whole skeleton of the human subject. X-ray images indicating myeloma may show metastases to the skeleton such as osteolytic changes, generalised osteopenia, or compressed vertebra. The bone marrow sample from the human subject indicating myeloma may show elevated levels of plasma cells with pathological morphology. Establishing the myeloma diagnosis may include further steps deemed necessary by a clinician or organisation.

If a myeloma diagnosis is established, step c) of the method of the fourth aspect may further comprise treating the human subject for myeloma.

Treating the human subject for myeloma may include steps such as administration of cytostatic drugs, radiation therapy, immunotherapy treatment, a stem cell transplant, CAR-T 19 treatment, and/or further steps deemed necessary by a clinician.

As a fifth aspect of the present disclosure, there is provided a method of detecting an increased risk of having or developing colorectal cancer, comprising the steps of:
a) determining the levels of at least proteins PADI4, TFRC, and PRDX5 in a blood sample from a human subject;
b) comparing the determined levels to reference levels; and
c) if the determined levels are higher than the reference levels, concluding that the human subject has the increased risk of having or developing colorectal cancer.

In one embodiment, step a) of the fifth aspect further comprises determining the level of at least one (such as at least two) of the proteins FKBP1B, PRDX6, LGALS4, CCL20, SELE, LAP3, and AREG. Accordingly, the determined level of FKBP1B, PRDX6, LGALS4, CCL20, SELE, LAP3 and/or AREG is compared to a reference level in step b) of this embodiment.

The fifth aspect can be carried out *ex vivo.*

The reference levels are preferably predetermined in human subjects not suffering from colorectal cancer, such as healthy human subjects and/or human subjects suffering from another cancer than colorectal cancer.

The blood sample may for example be a blood plasma sample or a sample derived from dried blood spots.

In one embodiment, step a) of the method of the fifth aspect comprises contacting the blood sample with:
an antibody, antibody fragment or aptamer selective for PADI4;
an antibody, antibody fragment or aptamer selective for TFRC; and
an antibody, antibody fragment or aptamer selective for PRDX5.

In one embodiment, step a) of the method of the fifth aspect may further comprise contacting the blood sample with:
an antibody, antibody fragment or aptamer selective for FKBP1B;
an antibody, antibody fragment or aptamer selective for PRDX6;
an antibody, antibody fragment or aptamer selective for LGALS4;
an antibody, antibody fragment or aptamer selective for CCL20;
an antibody, antibody fragment or aptamer selective for SELE;
an antibody, antibody fragment or aptamer selective for LAP3; and/or
an antibody, antibody fragment or aptamer selective for AREG.

In one embodiment of the fifth aspect, step c) further comprises a further examination of the human subject to establish a colorectal cancer diagnosis. The further examination comprises at least one of a proctoscopy on the human subject, a rectoscopy on the human subject, a rectal palpation on the human subject, or an examination of a tissue sample from a tumour-biopsy on the human subject.

Establishing a colorectal cancer diagnosis may further include a clinical examination of the human subject including assessment of the general condition of the human subject, wherein symptoms may include anemia, bleeding from the colon, and/or pain in the abdomen. A colorectal cancer diagnosis may be indicated by a rectal palpation and rectoscopy, in which case the human subject will undergo further examination. Tissue samples are collected and examined from identified tumours on the human subject. Establishing a colorectal cancer diagnosis may include following an individual examination plan provided by a clinician or organisation.

If a colorectal cancer diagnosis is established, step c) of the method of the fifth aspect may further comprise treating the human subject for colorectal cancer.

Treating the human subject for colorectal cancer may include steps such as administration of cytostatic drugs, immunotherapy treatment, surgical removal of tumour(s), and/or further steps deemed necessary by a clinician.

As a sixth aspect of the present disclosure, there is provided a method of detecting an increased risk of having or developing lung cancer, comprising the steps of:
a) determining the levels of at least proteins BCL2L11, MMP12, and CEACAM5 in a blood sample from a human subject;
b) comparing the determined levels to reference levels; and
c) if the determined levels are higher than the reference levels, concluding that the human subject has the increased risk of having or developing lung cancer.

In one embodiment, step a) of the sixth aspect further comprises determining the level of at least one (such as at least two) of the proteins PRDX5, LSM1, BPIFB1, ABHD14B, CXCL17, MLN, ANXA11, SFTPD, MTPN, SCGB3A2, LBP, ACP5, TFPI2, and COL9A1. Accordingly, the determined level of PRDX5, LSM1, BPIFB1, ABHD14B, CXCL17, MLN, ANXA11, SFTPD, MTPN, SCGB3A2, LBP, ACP5, TFPI2, and/or COL9A1 is compared to a reference level in step b) of this embodiment.

The sixth aspect can be carried out *ex vivo.*

The reference levels are preferably predetermined in human subjects not suffering from lung cancer, such as healthy human subjects and/or human subjects suffering from another cancer than lung cancer.

The blood sample may for example be a blood plasma sample or a sample derived from dried blood spots.

In one embodiment, step a) of the method of the sixth aspect comprises contacting the blood sample with:
an antibody, antibody fragment or aptamer selective for BCL2L11;
an antibody, antibody fragment or aptamer selective for MMP12; and
an antibody, antibody fragment or aptamer selective for CEACAM5.

In one embodiment, step a) of the method of the sixth aspect may further comprise contacting the blood sample with:
an antibody, antibody fragment or aptamer selective for PRDX5;
an antibody, antibody fragment or aptamer selective for LSMi;
an antibody, antibody fragment or aptamer selective for BPIFB1;
an antibody, antibody fragment or aptamer selective for ABHD14B;
an antibody, antibody fragment or aptamer selective for CXCL17;
an antibody, antibody fragment or aptamer selective for MLN;
an antibody, antibody fragment or aptamer selective for ANXA11;
an antibody, antibody fragment or aptamer selective for SFTPD;
an antibody, antibody fragment or aptamer selective for MTPN;
an antibody, antibody fragment or aptamer selective for SCGB3A2;
an antibody, antibody fragment or aptamer selective for LBP;
an antibody, antibody fragment or aptamer selective for ACP5;
an antibody, antibody fragment or aptamer selective for TFPI2; and/or
an antibody, antibody fragment or aptamer selective for COL9A1.

In one embodiment of the sixth aspect, step c) further comprises a further examination of the human subject to establish a lung cancer diagnosis. The further examination comprises at least one of a fiberoptic bronchoscopy on the human subject, an examination of a tissue sample obtained by a tumour-biopsy on the human subject, an examination of at least one image obtained by a CT or PET scan of the lung of the human subject, or an examination of at least one image obtained by an endoscopic ultrasound on the human subject.

Establishing a lung cancer diagnosis may further include a clinical examination of the human subject including assessment of the general condition of the human subject, wherein symptoms may include coughing, dyspnea, or pain in the thorax. The assessment of the general condition may be categorized on a scale of performance status of the human subject. A fiberoptic bronchoscopy is performed to find tumours located in the lungs. If the tumor is peripheral a biopsy may be performed. Establishing a lung cancer diagnosis may include following an individual examination plan provided by a clinician or organisation.

If a lung cancer diagnosis is established, step c) of the method of the sixth aspect may further comprise treating the human subject for lung cancer.

Treating the human subject for lung cancer may include steps such as administration of cytostatic drugs, immunotherapy treatment, radiation therapy, surgical removal of tumour(s), and/or further steps deemed necessary by a clinician.

As a seventh aspect of the present disclosure, there is provided a method of detecting an increased risk of having or developing glioma, comprising the steps of:
a) determining the levels of at least proteins GFAP, BCAN, and ADAMTS13 in a blood sample from a human subject;
b) comparing the determined levels to reference levels; and
c) if the determined levels are higher than the reference levels, concluding that the human subject has the increased risk of having or developing glioma.

The seventh aspect can be carried out *ex vivo.*

The reference levels are preferably predetermined in human subjects not suffering from glioma, such as healthy human subjects and/or human subjects suffering from another cancer than glioma.

The blood sample may for example be a blood plasma sample or a sample derived from dried blood spots.

In one embodiment, step a) of the method of the seventh aspect comprises contacting the blood sample with:
an antibody, antibody fragment or aptamer selective for GFAP;
an antibody, antibody fragment or aptamer selective for BCAN; and
an antibody, antibody fragment or aptamer selective for ADAMTS13.

In one embodiment of the seventh aspect, step c) further comprises a further examination of the human subject to establish a glioma diagnosis. The further examination comprises an examination of at least one image obtained by a CT scan of the brain of the human subject.

Establishing a glioma diagnosis may include a first step of performing a clinical examination of the human subject including assessment of the general condition of the human subject, wherein the main symptom may be headaches. Tumour(s) may be detected by CT and/or MRT scan of the brain of the subject. Establishing a glioma diagnosis may include further steps deemed necessary by a clinician or organisation.

If a glioma diagnosis is established, step c) of the method of the seventh aspect may further comprise treating the human subject for glioma.

Treating the human subject for glioma may include steps such as removal of tumour(s), gamma knife surgery, cytostatic drugs, radiation therapy, and/or further steps deemed necessary by a clinician.

As a eighth aspect of the present disclosure, there is provided a method of detecting an increased risk of having or developing breast cancer, comprising the steps of:
a) determining the levels of at least proteins PRTG, LAMP3, and OXT in a blood sample from a human subject;
b) comparing the determined levels to reference levels; and
c) if the determined levels are higher than the reference levels, concluding that the human subject has the increased risk of having or developing breast cancer.

In one embodiment, step a) of the eighth aspect further comprises determining the level of at least one (such as at least two) of the proteins SDC4, HSD11B1, BTC, LPL, MSMB, and GLO1. Accordingly, the determined level of SDC4, HSD11B1, BTC, LPL, MSMB, and/or GLO1 is compared to a reference level in step b) of this embodiment.

The eighth aspect can be carried out *ex vivo.*

The reference levels are preferably predetermined in human subjects not suffering from breast cancer, such as healthy human subjects and/or human subjects suffering from another cancer than breast cancer.

The blood sample may for example be a blood plasma sample or a sample derived from dried blood spots.

In one embodiment, step a) of the method of the eighth aspect comprises contacting the blood sample with:
an antibody, antibody fragment or aptamer selective for PRTG;
an antibody, antibody fragment or aptamer selective for LAMP3; and
an antibody, antibody fragment or aptamer selective for OXT.

In one embodiment, step a) of the method of the eighth aspect may further comprise contacting the blood sample with:
an antibody, antibody fragment or aptamer selective for SDC4;
an antibody, antibody fragment or aptamer selective for HSD11B1;
an antibody, antibody fragment or aptamer selective for BTC;
an antibody, antibody fragment or aptamer selective for LPL;
an antibody, antibody fragment or aptamer selective for MSMB; and/or
an antibody, antibody fragment or aptamer selective for GLO1.

In one embodiment of the eighth aspect, step c) further comprises a further examination of the human subject to establish a breast cancer diagnosis. The further examination comprises an examination of at least one of a tissue sample from a tumour-biopsy on the human subject, or at least one image obtained by a mammography on the human subject.

Establishing a breast cancer diagnosis may further include a clinical examination of the human subject including assessment of the general condition and hereditary cancer patterns of the human subject. Symptoms indicative of breast cancer may include palpable tumours, changes in the breast such as swelling, retraction, asymmetries, and/or secretion from the nipple(s). Tumour(s) may be detected by examining at least one image obtained by a mammography on the human subject, and upon detecting the tumours a biopsy may be performed on the human subject. Establishing a breast cancer diagnosis may include further steps deemed necessary by a clinician or organisation.

If a breast cancer diagnosis is established, step c) of the method of the eighth aspect may further comprise treating the human subject for breast cancer.

Treating the human subject for breast cancer may include steps such as administration of cytostatic drugs, immunotherapy treatment, surgical removal of tumour(s) and surrounding tissue, radiation therapy, endocrine therapy, and/or further steps deemed necessary by a clinician.

As a ninth aspect of the present disclosure, there is provided a method of detecting an increased risk of having or developing cervical cancer, comprising the steps of:
a) determining the levels of at least proteins GLO1, CHRDL2, and FCGR3B in a blood sample from a human subject;
b) comparing the determined levels to reference levels; and
c) if the determined levels are higher than the reference levels, concluding that the human subject has the increased risk of having or developing cervical cancer.

In one embodiment, step a) of the ninth aspect further comprises determining the level of at least one (such as at least two) of the proteins CRNN, AGER, MFAP5, and LYPD3. Accordingly, the determined level of CRNN, AGER, MFAP5, and/or LYPD3 is compared to a reference level in step b) of this embodiment.

The ninth aspect can be carried out *ex vivo.*

The reference levels are preferably predetermined in human subjects not suffering from cervical cancer, such as healthy human subjects and/or human subjects suffering from another cancer than cervical cancer.

The blood sample may for example be a blood plasma sample or a sample derived from dried blood spots.

In one embodiment, step a) of the method of the ninth aspect comprises contacting the blood sample with:
an antibody, antibody fragment or aptamer selective for GLO1;
an antibody, antibody fragment or aptamer selective for CHRDL2; and
an antibody, antibody fragment or aptamer selective for FCGR3B.

In one embodiment, step a) of the method of the ninth aspect may further comprise contacting the blood sample with:
an antibody, antibody fragment or aptamer selective for CRNN;
an antibody, antibody fragment or aptamer selective for AGER;
an antibody, antibody fragment or aptamer selective for MFAP5; and/or
an antibody, antibody fragment or aptamer selective for LYPD3.

In one embodiment of the ninth aspect, step c) further comprises a further examination of the human subject to establish a cervical cancer diagnosis. The further examination comprises an examination of at least one of a tissue sample from the cervix of the human subject or a pap test from the human subject.

Establishing a cervical cancer diagnosis may further include a clinical examination of the human subject including assessment of the general condition of the human subject, wherein the main symptom may include postcoital or irregular bleeding from the vagina. The tissue samples from the human subject may be collected with or without anesthesia. Before treating the human subject, complementary images from a PET-CT scan or X-ray scan from the human subject may be examined.

If a cervical cancer diagnosis is established, step c) of the method of the ninth aspect may further comprise treating the human subject for cervical cancer.

Treating the human subject for cervical cancer may include steps such as administration of cytostatic drugs, immunotherapy treatment, surgical treatment, radiation therapy, and/or further steps deemed necessary by a clinician.

As a tenth aspect of the present disclosure, there is provided a method of detecting an increased risk of having or developing endometrial cancer, comprising the steps of:
a) determining the levels of at least proteins TNFSF10, PLAT, and DPT in a blood sample from a human subject;
b) comparing the determined levels to reference levels; and
c) if the determined levels are higher than the reference levels, concluding that the human subject has the increased risk of having or developing endometrial cancer.

In one embodiment, step a) of the tenth aspect further comprises determining the level of at least one (such as at least two) of the proteins CLEC7A, CLMP, and AFP. Accordingly, the determined level of CLEC7A, CLMP, and/or AFP is compared to a reference level in step b) of this embodiment.

The tenth aspect can be carried out *ex vivo.*

The reference levels are preferably predetermined in human subjects not suffering from endometrial cancer, such as healthy human subjects and/or human subjects suffering from another cancer than endometrial cancer.

The blood sample may for example be a blood plasma sample or a sample derived from dried blood spots.

In one embodiment, step a) of the method of the tenth aspect comprises contacting the blood sample with:
an antibody, antibody fragment or aptamer selective for TNFSF10;
an antibody, antibody fragment or aptamer selective for PLAT; and
an antibody, antibody fragment or aptamer selective for DPT.

In one embodiment, step a) of the method of the tenth aspect may further comprise contacting the blood sample with:
an antibody, antibody fragment or aptamer selective for CLEC7A;
an antibody, antibody fragment or aptamer selective for CLMP; and/or
an antibody, antibody fragment or aptamer selective for AFP.

In one embodiment of the tenth aspect, step c) further comprises a further examination of the human subject to establish an endometrial cancer diagnosis. The further examination comprises an examination of at least one of a tissue sample from the endometrium of the human subject, or a tissue sample obtained by scraping the uterus and/or cervix of the human subject.

Establishing an endometrial cancer diagnosis may further include a clinical examination of the human subject including assessment of the general condition and hereditary cancer patterns of the human subject. Symptoms may include irregular bleeding from the vagina and/or abdominal pain. A first step in establishing an endometrial diagnosis in a human subject may include measuring the thickness of the endometrium with ultrasound. If the endometrium is found to be thickened, a tissue sample from the endometrium, cervix, and/or uterus of the human subject is collected and examined. Establishing an endometrial cancer diagnosis may include further steps deemed necessary by a clinician or organisation.

If an endometrial cancer diagnosis is established, step c) of the method of the tenth aspect may further comprise treating the human subject for endometrial cancer.

Treating the human subject for cervical cancer may include steps such as administration of cytostatic drugs, surgical treatment, radiation therapy, hormonal treatment, and/or further steps deemed necessary by a clinician.

As a eleventh aspect of the present disclosure, there is provided a method of detecting an increased risk of having or developing ovarian cancer, comprising the steps of:
a) determining the levels of at least proteins PAEP, CDH3, and SSC5D in a blood sample from a human subject;
b) comparing the determined levels to reference levels; and
c) if the determined levels are higher than the reference levels, concluding that the human subject has the increased risk of having or developing ovarian cancer.

The eleventh aspect can be carried out *ex vivo.*

The reference levels are preferably predetermined in human subjects not suffering from ovarian cancer, such as healthy human subjects and/or human subjects suffering from another cancer than ovarian cancer.

The blood sample may for example be a blood plasma sample or a sample derived from dried blood spots.

In one embodiment, step a) of the method of the eleventh aspect comprises contacting the blood sample with:
an antibody, antibody fragment or aptamer selective for PAEP;
an antibody, antibody fragment or aptamer selective for CDH3; and
an antibody, antibody fragment or aptamer selective for SSC5D.

In one embodiment of the eleventh aspect, step c) further comprises a further examination of the human subject to establish an ovarian cancer diagnosis. The further examination comprises an examination of at least one image obtained from a pelvic ultrasound of the human subject, a second blood sample from the human subject, or at least one image obtained by a CT scan of the human subject.

Establishing an ovarian cancer diagnosis may further include a clinical examination of the human subject including assessment of the general condition and hereditary cancer patterns of the human subject. Symptoms indicative of ovarian cancer may include ascites, pain in the abdomen, swollen abdomen, changes in bowel habits, and/or a frequent need to urinate. A first step of establishing an ovarian cancer diagnosis may palpation of the abdomen and a gynaecological examination, after which the human subject will undergo further examination such as an pelvic ultrasound or examination of CT-images. Establishing an endometrial cancer diagnosis may include further steps deemed necessary by a clinician or organisation.

If an ovarian cancer diagnosis is established, step c) of the method of the eleventh aspect may further comprise treating the human subject for ovarian cancer.

Treating the human subject for ovarian cancer may include steps such as administration of cytostatic drugs, surgical treatment, and/or further steps deemed necessary by a clinician.

As a twelfth aspect of the present disclosure, there is provided a method of detecting an increased risk of having or developing prostate cancer, comprising the steps of:
a) determining the levels of at least proteins FAP, DNER, and IL20 in a blood sample from a human subject;
b) comparing the determined levels to reference levels; and
c) if the determined levels are higher than the reference levels, concluding that the human subject has the increased risk of having or developing prostate cancer.

In one embodiment, step a) of the twelfth aspect further comprises determining the level of at least one (such as at least two) of the proteins CXCL6, CD34, CDH17, CRTAC1, IL18RAP, TRAF2, ADAMTS8, GZMB, SPINK5, F3, and ICAM4. Accordingly, the determined level of CXCL6, CD34, CDH17, CRTAC1, IL18RAP, TRAF2, ADAMTS8, GZMB, SPINK5, F3, and/or ICAM4 is compared to a reference level in step b) of this embodiment.

The twelfth aspect can be carried out *ex vivo.*

The reference levels are preferably predetermined in human subjects not suffering from prostate cancer, such as healthy human subjects and/or human subjects suffering from another cancer than prostate cancer.

The blood sample may for example be a blood plasma sample or a sample derived from dried blood spots.

In one embodiment, step a) of the method of the twelfth aspect comprises contacting the blood sample with:
an antibody, antibody fragment or aptamer selective for FAP;
an antibody, antibody fragment or aptamer selective for DNER; and
an antibody, antibody fragment or aptamer selective for IL20.

In one embodiment, step a) of the method of the twelfth aspect may further comprise contacting the blood sample with:
an antibody, antibody fragment or aptamer selective for CXCL6;
an antibody, antibody fragment or aptamer selective for CD34;
an antibody, antibody fragment or aptamer selective for CDH17;
an antibody, antibody fragment or aptamer selective for CRTAC1;
an antibody, antibody fragment or aptamer selective for IL18RAP;
an antibody, antibody fragment or aptamer selective for TRAF2;
an antibody, antibody fragment or aptamer selective for ADAMTS8;
an antibody, antibody fragment or aptamer selective for GZMB;
an antibody, antibody fragment or aptamer selective for SPINK5;
an antibody, antibody fragment or aptamer selective for F3; and/or
an antibody, antibody fragment or aptamer selective for ICAM4;

In one embodiment of the twelfth aspect, step c) further comprises a further examination of the human subject to establish a prostate cancer diagnosis. The further examination comprises an examination of at least one of a prostate-specific antigen (PSA) test from the human subject, at least one image obtained by a magnetic resonance tomography on the prostate of the human subject, a tissue sample from a tumour-biopsy on the human subject.

Establishing a prostate cancer diagnosis may further include a clinical examination of the human subject including assessment of the general condition of the human subject, wherein symptoms may include anemia, hypercalcemia, loss of appetite, edema, or kidney failure. A prostate cancer diagnosis may be indicated by a palpation, in which case the human subject will undergo further examination, wherein the further examination may comprise following an individual examination plan provided by a clinician or organisation.

If a prostate cancer diagnosis is established, step c) of the method of the twelfth aspect may further comprise treating the human subject for prostate cancer.

Treating the human subject for prostate cancer may include steps such as administration of cytostatic drugs, surgical treatment, radiation therapy (such as brachytherapy), hormonal treatment, and/or further steps deemed necessary by a clinician.

### EXAMPLES

### Description of the study workflow

The plasma proteome of 1,477 cancer patients and 74 healthy individuals were characterized using the Olink Explore 1536 Proximity Extension Assay (PEA) technology, allowing the quantification of 1,463 proteins using less than 3 microliters of plasma (*13*). Aiming to identify a plasma proteome signature for each of the cancers, we devised a workflow based on AI-based prediction models and differential protein expression analysis **(****Fig. 1****).** We initially focused on proteins upregulated in one or several cancer types as compared to all other cancers, to minimize batch effects from sample collection and analysis. First, the overall proteome (1,463 proteins) was used as a predictor of disease outcome to identify proteins reflecting disease status of each cancer sample. Next, differential expression analysis allowed us to select a subset of proteins that were up-regulated in one cancer type compared to the other cancer types. Combining both results, we selected a set of relevant upregulated proteins for each cancer type and subsequently investigated whether a multiclassification model based on the selected proteins was able to distinguish between cancer types. We further validated the potential of the selected biomarkers by building cancer prediction models classifying each cancer from a healthy cohort, and finally confirmed that the classification allows for accurate identification of early-stage cancer patients.

### The pan-cancer cohort

In this study, we have characterized the plasma proteome of a pan-cancer cohort from the Uppsala-Umea Comprehensive Cancer Consortium (U-CAN) biobank (*15*), consisting of 1,477 patients from twelve cancer types, including acute myeloid leukemia (n= 50), chronic lymphocytic leukemia (n= 48), diffuse large B-cell lymphoma (n= 55), myeloma (n= 38), colorectal cancer (n= 221), lung cancer (n= 268), glioma (n= 145), breast cancer (n= 152), cervical cancer (n= 102), endometrial cancer (n= 101), ovarian cancer (n= 134) and prostate cancer (n= 163). Plasma samples were collected at the time of diagnosis and before treatment were initiated. Clinical meta data regarding age, sex, BMI, diagnosis, secondary diagnosis, and cancer stage or grade was available for the cancer samples. For each cancer, the age distribution is shown in **Fig. 2a****.**

### Identification of cancer-specific proteins

The plasma protein levels of 1,463 protein targets were determined for each of the 1,477 patients to generate more than 2 million data points representing individual plasma protein levels across all the 12 cancer types. The main aim was not to identify a single protein marker to uniquely identify each cancer, but instead to identify a protein signature from each of the cancers to aid in a pan-cancer identification. An initial analysis revealed several upregulated or downregulated proteins in specific cancer types as exemplified in **Fig. 2b****.**

### Pan-cancer prediction models

To identify proteins relevant for each cancer type, a disease prediction model was built for each cancer type (n=12), respectively, using all measured proteins (n=1,463) and 70% of the cancer patients as the training set. The control group in each model was composed of all the other cancer samples and was subsampled to include a similar number of patients to the modelled cancer. We compared the results obtained from two classification algorithms, random forest (RF) and a regularized generalized linear model (glmnet), both of which give an estimation of the overall importance of each protein to the model (range 0-100%). A heatmap visualization shows the importance score for the 486 proteins that scored high (>25% importance) in at least one of the cancer types by glmnet. We observed that some of the cancer models have a higher number of proteins with high importance scores, suggesting that more proteins are relevant for a correct classification. Moreover, several proteins scored high (>25% importance) in more than one cancer, as demonstrated in the network visualization revealing relationships between the potential biomarkers in the different cancer types (not shown).

Since two prediction models were used for the analysis (random forest and glmnet), we compared the importance scores for each of the 1,463 protein targets in each of the cancer types given by the two models **(****Fig. 2c****).** For some cancers (e.g. glioma), one protein is given the highest score by both models and with considerably lower scores for the other proteins (< 50%), while in other cancers there is a continuum of importance scores that can be mainly consistent (e.g. in myeloma) or not consistent (e.g. in endometrial cancer). In general, the two models predict the same proteins with similar importance, but the random forest models tend to be more conservative overall, consistently selecting fewer important proteins.

### Evaluation of cancer-specific prediction models

The performance of the cancer prediction models was evaluated using the 30% of the data excluded from the model training. In **Fig. 3a****,** the classification of all patients in the test cohort using both prediction algorithms were performed, scoring each plasma sample for the probability to come from a specific cancer type. We found that both models can efficiently separate samples from all the specific cancers, with particularly high confidence for the immune cell related cancers AML, CLL and myeloma. The two prediction models yield similar results, but since the glmnet results yielded slightly more accurate discrimination of the patients, we decided to continue the exploratory analysis using the glmnet algorithm.

### Characterization of differentially expressed proteins across the cancers

To further investigate the cancer-specific proteome profiles, differential expression analyses were performed in a setting where each cancer was compared to all other cancers. For the male and female cancers, only samples with the same sex were compared. The results showed a distinct differentially expressed proteome for each of the cancers. The differential analysis identified, as expected, similar potential biomarkers as the prediction models, exemplified by FLT3 for AML and CXCL17 and FKBP1 for lung cancer. However, some of the proteins, such as EPO in AML, show high significance in the differential analysis, but were not used by either the random forest or the glmnet prediction models.

In **Fig. 3b****,** the number of upregulated proteins that are shared by different cancer types is shown for the complete set of proteins analyzed. As expected, there were a large number of upregulated proteins shared by the four immune cell related cancers (AML, CLL, lymphoma and myeloma), in many cases consisting of proteins related to immune-related functions. However, most overlapping proteins were observed for lung and colorectal cancer. This surprising observation might reflect common features, mainly adenocarcinoma with a high fraction of high-grade tumors with likely similar host inflammatory response between these two cancer types. A functional gene ontology (GO) analysis was also performed on the up-regulated proteins for each of the cancer types. As expected, the up-regulated proteins in the immune cell related cancers (AML, CLL and lymphoma) are related to immune processes, while breast, endometrial and prostate cancer had an over-representation of cell adhesion proteins. Interestingly, both lung and colorectal cancer had an over-representation of apoptotic-related proteins. More in-depth analysis is needed to explore the biological and medical significance of the functional analysis for each of the up-regulated proteins.

### Selection of a panel with cancer-specific proteins

Combining the previous results, we sought to identify a panel of proteins based on the ranking from the glmnet models and relevant to each of the analyzed cancers. We only included proteins identified as upregulated by differential expression analysis, aiming to include at least three proteins per cancer and all proteins with more than 50% overall importance as indicated by the cancer prediction models. Based on these criteria, a panel of 83 proteins capturing cancer-specific profiles was selected **(****Fig. 4a****).** Lung- and prostate cancer contributed to the largest number of proteins in the panel, 18 and 14, respectively, whereas only three protein targets each were selected for AML, glioma, myeloma and ovarian cancer.

In **Fig. 4b****,** the average plasma levels of the 83 selected protein members of the panel are visualized across all cancer types. Most of the selected proteins had a higher level in only one cancer, while some had high protein levels in multiple cancers. For example, CXADR like membrane protein (CLM), selected to identify endometrial cancer, also showed elevated plasma levels in myeloma patients. Only two of the proteins were given a high importance score (>50%) by the prediction model in more than one cancer. Both FKB prolyl isomerase 1B (FKBP1B) and peroxiredoxin 5 (PRDX5) had higher plasma levels in lung- and colorectal cancer as compared to all the other cancers were also selected independently by the models for both of these cancer types **(****Fig. 4a****).** Interestingly, FKBP1B is involved in immunoregulation and protein folding and has previously been linked to colorectal cancer (*16*) but not to lung cancer. Similarly, PRDX5 has an antioxidant function in normal and inflammatory conditions and although not previously suggested to be involved in cancers, several other proteins of the peroxiredoxin family have been linked to lung and colorectal cancers in transcriptomics analysis of cancer cell lines (*17*,*18*).

### Classification of the pan-cancer cohort based on the selected protein panel

A multiclass classification based on the glmnet algorithm was used to explore the sensitivity and specificity of the panel proteins to predict a specific cancer and to assess the model's ability to distinguish the different cancer types. Comparative receiver operating characteristic (ROC) analyses were performed for each cancer type in which the specificity/sensitivity measured as area under the curve (AUC) (*19*) was determined for different number of proteins. These analyses included (i) all proteins (n=1,463), (ii) those selected in the panel (n=83), (iii) the three most important proteins per cancer and (iv) the single most important protein per cancer. The results **(****Fig. 5a****)** show that the panel of 83 proteins can identify the right cancer with both high selectivity and sensitivity with AUC ranging between 0.93 and for all cancer types. The analysis using all proteins gave only slightly better results. The use of only the top 3 proteins in each cancer also gave clinically relevant results even if not of the same significance. The performance scores obtained when using only the top (single) protein for each of the 12 cancers were generally considered to be too low. The AUC values for the different protein numbers are summarized for each of the cancers **(****Fig. 5b****),** showing the advantage of multiple proteins to identify cancer patients from blood plasma. However, the results also suggest that an AI-based prediction model can avoid the requirement of a proteome-wide analysis and instead select a smaller panel of proteins with almost the same prediction reliability as using all proteins. Here, our results demonstrate that a panel of only 83 proteins yields highly promising results (AUC) for simultaneous identification of all 12 cancer types. If only a single cancer type is to be identified, a three-protein panel suffice (even though a larger panel may provide a more accurate result).

As shown in fig. 5a-b, the top 3 AUC is 1 for AML, 1 for CLL, 0.91 for DLBCL, 0.99 for Myeloma, 0.91 for lung cancer, 0.93 for colorectal cancer, 0.97 for glioma, 0.92 for prostate cancer, 0.9 for breast cancer, 0.91 for cervical cancer, 0.91 for endometrial cancer and 0.91 for ovarian cancer.

### Performance of classification of cancer samples from a healthy cohort.

An important question is how well the model based on the pan-cancer study can distinguish cancer patients from healthy individuals. To investigate this, for each of the 12 cancer types, a new cancer prediction model was built but this time including 74 healthy individuals previously studied as part of a wellness study (*20*, *21*) as the control group instead of all of the other cancers. As described above, each of the cancers contributed to the panel with a different number of proteins (3-18) and these new models were based only on these specific proteins. We again used 70% of the samples as the training set and the remaining 30% to test the performance of the model. The results for four of the cancers are shown in **Fig. 6a****-d** and all cancers in **Fig. 6h****.** For CLL **(****Fig. 6a****),** the model can distinguish cancer patients from healthy controls with total accuracy (AUC=1). Similarly, the same analysis for colorectal-**(****Fig. 6b****),** ovarian- **(****Fig. 6c****)** and lung cancer **(****Fig. 6d****),** respectively, shows high accuracy with all AUC results above 0.83, demonstrating that the protein panel can distinguish cancer patients from healthy individuals with high accuracy. These results suggest that the protein panel and the prediction model are suitable to identify patients with the analyzed cancer types as well as distinguish cancer patients from healthy individuals (without a cancer diagnosis).

### Patients with cancers of different stages

An important quest in the field of Cancer Precision Medicine is to aid clinicians to indicate the stage of the cancer. For some cancers in this study, a relatively large number of patients had stage data available and therefore we investigated whether the protein panel could stratify patients into stages for these cancer types. In **Fig. 6e****,** show four examples of proteins where the plasma levels correlate with disease stage, including (i) CD22 used to identify CLL patients; (ii) amphiregulin (AREG) in colorectal cancer patients; (iii) arbhydrolsase domain containing 14B (ABHD14B) in lung cancer patients; and (iv) the ovarian cancer biomarker Progestagen associated endometrial protein (PAEP). For all these examples, the protein levels in healthy individuals (wellness) are lower than protein levels in all stages of each cancer. These examples further demonstrate the possibility to perform stage stratification simply by analysing selected plasma protein levels.

### Classification of early-stage cancer samples

The most important objective in the field of cancer precision medicine is to identify cancer at an early stage to provide successful therapeutic intervention and patient survival. To assess the ability of the protein panel to distinguish early-stage cancer from healthy individuals, we focused on patients with early stage colorectal and lung cancer, where the sample sizes of patients with less advanced disease (stage 1) are relatively large. In **Fig. 6g****,** the cancer probability score for early colorectal cancer patients (stage 1) is compared with the corresponding score for healthy individuals (wellness). A clear difference in score is shown for most samples and the AUC-score **(****Fig. 6g****)** for separating stage colorectal patients from healthy individuals is 0.78. Similarly, for the early lung cancer patients a clear difference is predicted by the protein panel model **(****Fig. 6f****),** although the AUC-score **(****Fig. 6f****)** is somewhat lower (0.79). This highlights the potential of the selected biomarker panel to identify early-stage cancers.

### REFERENCES

1. D. Crosby et al., Early detection of cancer. Science 375, eaay9040 (2022).
2. E. Surveillance, and End Results (SEER) Program.
3. D. Ilic et al., Prostate cancer screening with prostate-specific antigen (PSA) test: a systematic review and meta-analysis. BMJ 362, k3519 (2018).
4. U. Ladabaum, J. A. Dominitz, C. Kahi, R. E. Schoen, Strategies for Colorectal Cancer Screening. Gastroenterology 158, 418-432 (2020).
5. A. Yala et al., Optimizing risk-based breast cancer screening policies with reinforcement learning. Nat Med 28, 136-143 (2022).
6. N. Cancer Genome Atlas Research et al., The Cancer Genome Atlas Pan-Cancer analysis project. Nat Genet 45, 1113-1120 (2013).
7. C. Hutter, J. C. Zenklusen, The Cancer Genome Atlas: Creating Lasting Value beyond Its Data. Cell 173, 283-285 (2018).
8. J. Zhang et al., The International Cancer Genome Consortium Data Portal. Nat Biotechnol 37, 367-369 (2019).
9. I. T. P.-C. A. o. W. G. Consortium, Pan-cancer analysis of whole genomes. Nature 578, 82-93 (2020).
10. A. A. Friedman, A. Letai, D. E. Fisher, K. T. Flaherty, Precision medicine for cancer with next-generation functional diagnostics. Nat Rev Cancer 15, 747-756 (2015).
11. R. Akbani et al., A pan-cancer proteomic perspective on The Cancer Genome Atlas. Nat Commun 5, 3887 (2014).
12. L. Gold, J. J. Walker, S. K. Wilcox, S. Williams, Advances in human proteomics at high scale with the SOMAscan proteomics platform. N Biotechnol 29, 543-549 (2012).
13. L. Wik et al., Proximity Extension Assay in Combination with Next-Generation Sequencing for High-throughput Proteome-wide Analysis. Mol Cell Proteomics 20, 100168 (2021).
14. W. Zhong et al., Next generation plasma proteome profiling to monitor health and disease. Nat Commun 12, 2493 (2021).
15. B. Glimelius et al., U-CAN: a prospective longitudinal collection of biomaterials and clinical information from adult cancer patients in Sweden. Acta Oncol 57, 187-194 (2018).
16. L. Wang, X. Jiang, X. Zhang, P. Shu, Prognostic implications of an autophagy-based signature in colorectal cancer. Medicine (Baltimore) 100, e25148 (2021).
17. M. K. Kim et al., Patients with ERCC1-negative locally advanced esophageal cancers may benefit from preoperative chemoradiotherapy. Clin Cancer Res 14, 4225-4231 (2008).
18. W. Lu et al., Peroxiredoxin 2 is upregulated in colorectal cancer and contributes to colorectal cancer cells' survival by protecting cells from oxidative stress. Mol Cell Biochem 387, 261-270 (2014).
19. F. T., An introduction to ROC analysis. Pattern Recognition Letters 27, 861-874 (2006).
20. G. Bergstrom et al., The Swedish CArdioPulmonary BioImage Study: objectives and design. J Intern Med 278, 645-659 (2015).
21. A. Tebani et al., Integration of molecular profiles in a longitudinal wellness profiling cohort. Nat Commun 11, 4487 (2020).
22. C. B. Holst et al., Plasma IL-8 and ICOSLG as prognostic biomarkers in glioblastoma. Neurooncol Adv 3, vdab072 (2021).
23. H. Jaksch-Bogensperger et al., Proseek single-plex protein assay kit system to detect sAxl and Gas6 in serological material of brain tumor patients. Biotechnol Rep (Amst) 18, e00252 (2018).
24. E. Engvall, P. Perlmann, Enzyme-linked immunosorbent assay, Elisa. 3. Quantitation of specific antibodies by enzyme-labeled anti-immunoglobulin in antigen-coated tubes. J Immunol 109, 129-135 (1972).

## Claims

1. A method of detecting an increased risk of having or developing colorectal cancer, comprising the steps of:
a) determining the levels of at least proteins PADI4, TFRC, and PRDX5 in a blood sample from a human subject;
b) comparing the determined levels to reference levels; and
c) if the determined levels are higher than the reference levels, concluding that the human subject has the increased risk of having or developing colorectal cancer.

2. The method of claim 1, wherein step a) comprises contacting the blood sample with at least:
an antibody, antibody fragment or aptamer selective for PADI4;
an antibody, antibody fragment or aptamer selective for TFRC; and
an antibody, antibody fragment or aptamer selective for PRDX5.

3. The method of claim or 2, wherein step a) further comprises determining the level of at least one of the proteins FKBP1B, PRDX6, LGALS4, CCL20, SELE, LAP3, and AREG.

4. The method of any one of the preceding claims, wherein step a) further comprises determining the levels of at least two of the proteins FKBP1B, PRDX6, LGALS4, CCL20, SELE, LAP3, and AREG.

5. The method of any one of the preceding claims, wherein step a) further comprises determining the levels of at least five of the proteins FKBP1B, PRDX6, LGALS4, CCL20, SELE, LAP3, and AREG.

6. The method of any one of claims 3-5, wherein step a) further comprises contacting the blood sample with:
an antibody, antibody fragment or aptamer selective for FKBP1B;
an antibody, antibody fragment or aptamer selective for PRDX6;
an antibody, antibody fragment or aptamer selective for LGALS4;
an antibody, antibody fragment or aptamer selective for CCL20;
an antibody, antibody fragment or aptamer selective for SELE;
an antibody, antibody fragment or aptamer selective for LAP3; and/or
an antibody, antibody fragment or aptamer selective for AREG.

7. The method of any one of the preceding claims, wherein the reference levels are predetermined in human subjects not suffering from colorectal cancer, such as healthy human subjects and/or human subjects suffering from another cancer than colorectal cancer.

8. The method of any one of the preceding claims, wherein the blood sample is a blood plasma sample or a sample derived from dried blood spots.

9. A method of detecting an increased risk of having or developing lung cancer, comprising the steps of:
a) determining the levels of at least proteins BCL2L11, MMP12, and CEACAM5 in a blood sample from a human subject;
b) comparing the determined levels to reference levels; and
c) if the determined levels are higher than the reference levels, concluding that the human subject has the increased risk of having or developing lung cancer.

10. The method of claim 9, wherein step a) comprises contacting the blood sample with at least:
an antibody, antibody fragment or aptamer selective for BCL2L11;
an antibody, antibody fragment or aptamer selective for MMP12; and
an antibody, antibody fragment or aptamer selective for CEACAM5.

11. A method of detecting an increased risk of having or developing breast cancer, comprising the steps of:
a) determining the levels of at least proteins PRTG, LAMP3, and OXT in a blood sample from a human subject;
b) comparing the determined levels to reference levels; and
c) if the determined levels are higher than the reference levels, concluding that the human subject has the increased risk of having or developing breast cancer.

12. The method of claim 11, wherein step a) comprises contacting the blood sample with at least:
an antibody, antibody fragment or aptamer selective for PRTG;
an antibody, antibody fragment or aptamer selective for LAMP3; and
an antibody, antibody fragment or aptamer selective for OXT.

13. A method of detecting an increased risk of having or developing prostate cancer, comprising the steps of:
a) determining the levels of at least proteins FAP, DNER, and IL20 in a blood sample from a human subject;
b) comparing the determined levels to reference levels; and
c) if the determined levels are higher than the reference levels, concluding that the human subject has the increased risk of having or developing prostate cancer.

14. The method of claim 13, wherein step a) comprises contacting the blood sample with at least:
an antibody, antibody fragment or aptamer selective for FAP;
an antibody, antibody fragment or aptamer selective for DNER; and
an antibody, antibody fragment or aptamer selective for IL20.

15. A method of detecting an increased risk of having or developing acute myeloid leukemia (AML), comprising the steps of:
a) determining the levels of at least proteins CD244, TNFSF13B , and FLT3 in a blood sample from a human subject;
b) comparing the determined levels to reference levels; and
c) if the determined levels are higher than the reference levels, concluding that the human subject has the increased risk of having or developing AML.
